# EUROPÄISCHE PATENTSCHRIFT

(11) **EP 4 312 920 B1**
(45) Veröffentlichungstag und Bekanntmachung des Hinweises auf die Patenterteilung: **12.08.2026**
(21) Anmeldenummer: 22713931.8
(22) Anmeldetag: 11.03.2022
(51) Int. Cl.: A61F 9/008, A61F 9/009

(54) **VERFAHREN ZUR DURCHFÜHRUNG EINES SYSTEMTESTS EINES LASERBEARBEITUNGSSYSTEMS, STEUEREINHEIT UND LASERBEARBEITUNGSSYSTEM**
METHOD FOR CARRYING OUT A SYSTEM TEST OF A LASER PROCESSING SYSTEM, CONTROL UNIT AND LASER PROCESSING SYSTEM
PROCÉDÉ DE RÉALISATION DE TEST DE SYSTÈME DE TRAITEMENT LASER, UNITÉ DE COMMANDE ET SYSTÈME DE TRAITEMENT LASER

(30) Priorität: 25.03.2021 DE 102021202947
(43) Veröffentlichungstag der Anmeldung: 07.02.2024
(73) Patentinhaber: Carl Zeiss Meditec AG, 07745 Jena (DE)
(72) Erfinder: WEIMER, Wolf, 07745 Jena (DE); STOBRAWA, Gregor, 07745 Jena (DE); BISCHOFF, Mark, 07745 Jena (DE)
(74) Vertreter: Pearl Cohen EU
(86) Internationale Anmeldenummer: PCT/EP2022/056281
(87) Internationale Veröffentlichungsnummer: WO 2022/200072

(56) Entgegenhaltungen:
- WO-A1-2012/076031
- US-A1- 2002 193 704
- US-A1- 2007 173 792

## Beschreibung

Die vorliegende Erfindung betrifft ein Verfahren zur Durchführung eines Systemtests eines Laserbearbeitungssystems, eine Steuereinheit und ein Laserbearbeitungssystem. Die Erfindung liegt somit insbesondere auf dem Gebiet der Laserbearbeitungssysteme und insbesondere auf dem Gebiet der ophthalmologischen Laserbearbeitungssysteme für die Augenchirurgie.

Ophthalmologische Laserbearbeitungssysteme sind Therapiesysteme für die Augenchirurgie. Ihre einwandfreie Funktion ist Voraussetzung für den Therapieerfolg sowie für die Sicherheit des Patienten und des Anwenders. Oftmals erfordert dies eine automatische oder halbautomatische Überprüfung bestimmter Eigenschaften der Laserbearbeitungssysteme vor einer Behandlung.

Die einwandfreie Funktion kann auch ein Erfordernis des Erreichens einer bestimmten Genauigkeit bei der Therapie beinhalten. Zu diesem Zweck ist es mitunter vorteilhaft, dass gezielte Maßnahmen zur Genauigkeitsverbesserung durchgeführt werden. Dies ist insbesondere dann der Fall, wenn Veränderungen am Laserbearbeitungssystem eine Kalibrierung erforderlich machen. Solche Veränderungen können beispielsweise durch äußere Einflüsse hervorgerufen werden. In besonderem Maße treten solche Veränderungen dann auf, wenn ein Laserbearbeitungssystem mit anderen Medizingeräten kombiniert wird bzw. selbst ein kombinierbares Zubehör beinhaltet. So kann beispielsweise die Verwendung eines Patienteninterfaces in Form eines sterilen Verbrauchsmaterials eine Variabilität im System erzeugen, die eine diese Variabilität ausgleichende Kalibrierung notwendig macht.

Oft werden also vor einer Behandlung eines Auges mittels eines Laserbearbeitungssystems ein oder mehrere Testverfahren und/oder Systemtests, welche Tests und/oder Kalibrierungen umfassen können, angewendet, um sicherzustellen, dass das Laserbearbeitungssystem seine Funktion sicher und wirksam erfüllt. Die einzelnen Tests und/oder Kalibrierungen, die ein Systemtest umfasst, werden dabei sequenziell nacheinander durchgeführt.

Ein Systemtest kann beispielsweise dann eine Kalibrierung umfassen, wenn die Benutzung des Laserbearbeitungssystems die Verwendung von austauschbaren Kontaktgläsern beinhaltet, die möglicherweise aufgrund von Fertigungstoleranzen in ihrer Stärke, ihrem Durchmesser, ihrer Dezentrierung (lateralem Versatz relativ zur optischen Achse), ihrer Form (Krümmungsradius) und/oder hinsichtlich anderer Eigenschaften variieren können. Für die Verbesserung der erreichbaren Präzision des Laserbearbeitungssystems und einer damit durchzuführenden Behandlung können die ermittelten Parameter bzw. die Ergebnisse eines Systemtest zur Optimierung der Steuersignale für die Scaneinrichtung verwendet werden.

Es gibt verschiedene Möglichkeiten zur zeitlichen Anordnung eines Systemtest bzw. der einzelnen Tests und/oder Kalibrierungen eines Systemtests, beispielsweise gleich nach dem Einschalten des Laserbearbeitungssystems und/oder unmittelbar vor einer Therapie. Es ist vorteilhaft, alle Tests und/oder Kalibrierungen des Systemtests unmittelbar vor der Verwendung des Laserbearbeitungssystems zur Behandlung eines Auges durchzuführen, um die Zeitspanne zwischen den jeweiligen Tests und/oder Kalibrierungen und der Behandlung, in welcher unerwünschte Veränderungen auftreten können, zu minimieren. Solche unerwünschten Veränderungen können etwa durch thermische Drifts, spontane Komponentenfehler, mechanische Veränderungen oder Änderungen von Einstellungen durch ein versehentliches Berühren und/oder ein Kontaminieren des Kontaktglases und/oder anderer Komponenten hervorgerufen werden. Eine Durchführung aller Tests und/oder Kalibrierungen des Systemtests unmittelbar vor der Durchführung einer Behandlung erfordert jedoch herkömmlicherweise, dass sich der Patient während der gesamten Dauer der Durchführung des Systemtests in behandlungsbereitem Zustand befindet. Dies kann erfordern, dass sich der Patient während der gesamten Zeitspanne des Systemtests in einer unerwünschten Zwangslage befindet und dabei ggf. OP-Kleidung und/oder eine Lidsperre tragen muss, medikamentiert sein muss und/oder am Laserbearbeitungsgerät, insbesondere am Kontaktglas, angedockt verweilen muss. Dies kann sich bei erheblicher Zeitdauer des Systemtests nachteilhaft auf das Wohlempfinden des Patienten auswirken oder gar Stress verursachen.

Die WO2012/076031A1 beschreibt eine Lasereinrichtung für die ophthalmische Chirurgie, welche dazu eingerichtet ist, bei abgeschalteter Laserquelle einen Testbetriebslauf durchzuführen, bei dem die Scankomponenten oder zumindest ein Teil derselben nach Maßgabe eines vorgegebenen Test-Scanmusters gesteuert werden. Dabei kann der tatsächliche Einstellzustand wenigstes eines Teils der Scankomponenten messtechnisch erfasst werden, um die Ist-Position des Strahlfokus zu berechnen.

Die US2002/193704A1 beschreibt eine Vorrichtung und ein System für die Photodisruption von Augengewebe. Dabei können eine Kalibration und optional eine zusätzliche Energiekalibration oder weitere Kalibrationsvorgänge durchgeführt werden.

Die US2007/173792A1 beschreibt Systeme und Vorrichtungen zum Testen eines Laser-Augenchirurgiesystems. Dabei wird ein Kalibrationsmuster abgebildet und zusätzlich kann das Strahlführungssystem hinsichtlich seiner lateralen Ablenkungscharakteristik kalibriert werden.

Es ist daher die Aufgabe der Erfindung, die erforderliche Zeitdauer für einen Systemtest eines Laserbearbeitungssystems zu reduzieren.

Diese Aufgabe wird durch ein Verfahren zur Durchführung eines Systemtests eines Laserbearbeitungssystems, eine Steuereinheit und ein Laserbearbeitungssystem mit den Merkmalen des jeweiligen unabhängigen Anspruchs gelöst. Vorteilhafte Ausführungsformen sind Gegenstand der abhängigen Ansprüche und der nachfolgenden Beschreibung.

In einem ersten Aspekt betrifft die Erfindung ein Verfahren zur Durchführung eines Systemtests eines Laserbearbeitungssystems nach Anspruch 1. Das Verfahren umfasst dabei ein Durchführen einer Funktionsprüfung eines Steuerelements des Laserbearbeitungssystems. Zudem umfasst das Verfahren ein Ermitteln eines variablen Parameters und Bestimmen eines Einflusses des ermittelten variablen Parameters auf eine beabsichtigte Durchführung einer Laserbearbeitung mittels des Laserbearbeitungssystems. Dabei überlappen das Durchführen der Funktionsprüfung des Steuerelements und das Ermitteln des variablen Parameters zumindest teilweise zeitlich einander.

In einem weiteren Aspekt betrifft die Erfindung eine Steuereinheit für ein Laserbearbeitungssystems nach Anspruch 14, wobei die Steuereinheit dazu eingerichtet ist, im Rahmen eines Systemtests des Laserbearbeitungssystems eine Funktionsprüfung des Steuerelements des Laserbearbeitungssystems durchzuführen. Außerdem ist die Steuereinheit dazu eingerichtet, einen variablen Parameter zu ermitteln und einen Einfluss des variablen Parameters auf eine beabsichtigte Durchführung einer Laserbearbeitung mittels des Laserbearbeitungssystems zu ermitteln. Ferner ist die Steuereinheit dazu eingerichtet, die Funktionsprüfung des Steuerelements und das Ermitteln des variablen Parameters zumindest teilweise zeitlich einander überlappend durchzuführen und daraus resultierende Messdaten und/oder Messergebnisse zumindest teilweise zeitgleich zu empfangen und/oder auszuwerten.

In einem weiteren Aspekt betrifft die Erfindung ein Laserbearbeitungssystem umfassend eine erfindungsgemäße Steuereinheit nach Anspruch 15.

Ein Systemtest ist dabei ein Verfahren zur Überprüfung und/oder Sicherstellung der Funktionalität des Laserbearbeitungssystems. Der Systemtest kann dabei eine oder mehrere Tests und/oder eine oder mehrere Kalibrierungen umfassen. Ein Systemtest kann in einem zusammenhängenden Zeitraum oder in mehreren voneinander beabstandeten Zeiträumen durchführbar sein. Ein Systemtest ist optional derart ausgestaltet, dass dieser automatisiert durchführbar ist, insbesondere durch das Laserbearbeitungssystem selbst. Jedoch sind gemäß manchen Ausführungsformen auch Systemtests möglich, welche unter Mitwirkung eines Bedieners durchführbar sind.

Ein Test stellt dabei eine Funktionsprüfung eines Steuerelements mittels mindestens eines Sensors dar. Eine Kalibrierung stellt eine Messung einer oder mehrerer Eigenschaften eines variablen Elements und/oder einer variablen Komponente unter Verwendung mindestens eines Sensors und die Ableitung einer Anpassung von zukünftigen Steuersignalen gemäß den ermittelten Messwerten dar.

Ein Laserbearbeitungssystem ist dabei ein System, welches die Bearbeitung eines Objektes mittels Laserstrahlung ermöglicht. Insbesondere kann ein Laserbearbeitungssystem als ophthalmisches Laserbearbeitungssystem ausgebildet sein und für die chirurgische Bearbeitung bzw. Behandlung eines menschlichen und/oder tierischen Auges eines Patienten dienen. Optional kann das Laserbearbeitungssystem als eine Vorrichtung für die Korrektur von Myopie oder Hyperopie und/oder Astigmatismus eines Auges ausgebildet sein. Insbesondere kann ein Laserbearbeitungssystem eine Laserquelle umfassen und/oder einen Laserstrahl von einer externen, separaten Laserquelle empfangen. Die Laserquelle umfasst vorzugsweise einen Femtosekunden-Laser und/oder einen Pikosekunden-Laser oder ist als solcher ausgebildet. Gemäß anderen Ausführungsformen kann die Laserquelle jedoch auch einen Excimer-Laser umfassen. Der Laserstrahl kann vorzugsweise als gepulster Laserstrahl, oder auch als Dauerstrich-Laserstrahl (cw) bereitgestellt werden.

Ein Steuerelement ist dabei ein Element, welches steuerbar und/oder regelbar ist. Beispielsweise kann das Steuerelement einen Aktor darstellen. Optional kann das Steuerelement als ein steuerbares optisches Element ausgebildet sein oder ein solches umfassen, wie etwa als ein adaptiver Spiegel und/oder als ein oder mehrere bewegliche Spiegel. Insbesondere kann ein Steuerelement dazu ausgelegt sein, den Laserstrahl des Laserbearbeitungssystems zu steuern, etwa durch ein gezieltes Ablenken des Laserstrahls. Beispielsweise kann das Steuerelement als ein Laserstrahl-Scanner, d.h. als Scaneinrichtung, ausgebildet sein, mittels welchem der Laserstrahl über einen oder mehrere bewegliche Spiegel in seiner Position und/oder Ausbreitungsrichtung angepasst werden kann.

Das Ermitteln eines variablen Parameters stellt ein Beschaffen von Informationen über den tatsächlichen Wert und/oder Zustand des variablen Parameters zumindest zum Zeitpunkt der Ermittlung dar. Die ermittelten Informationen bezüglich des variablen Parameters sind dabei repräsentativ für den veränderlichen Anteil des variablen Parameters. Beispielsweise kann es sich dabei um Informationen über die tatsächliche Ausgestaltung von Elementen und/oder Komponenten handeln, die Änderungen unterworfen sind. Alternativ oder zusätzlich kann es sich dabei um andere Größen handeln, die keine Eigenschaft des Laserbearbeitungssystems darstellen, wie etwa die Position und/oder Ausrichtung eines zu behandelnden Auges.

Dass das Durchführen der Funktionsprüfung des Steuerelements und das Ermitteln des variablen Parameters zumindest teilweise zeitlich überlappen, bedeutet, dass diese zeitlich nicht vollständig separat voneinander durchgeführt werden. Mit anderen Worten bedeutet dies, dass zu zumindest einem Zeitpunkt sowohl das Durchführen der Funktionsprüfung des Steuerelements als auch das Ermitteln des variablen Parameters erfolgen. Optional können beide Vorgänge auch vollständig überlappen, d.h. dass beide zum gleichen Zeitpunkt beginnen und/oder zum gleichen Zeitpunkt enden, oder das Durchführen der Funktionsprüfung des Steuerelements oder das Ermitteln des variablen Parameters dann beginnen und enden, während der jeweils andere Vorgang noch andauert.

Dass die Messdaten und/oder Messergebnisse zumindest teilweise zeitgleich von der Steuereinheit empfangen und/oder ausgewertet werden können, bedeutet dabei, dass die Recheneinheit nicht das Empfangen und/oder Auswerten der ersten Messdaten und/oder Messergebnisse abschließen muss, bevor zweite Messdaten und/oder Messergebnisse empfangen und/oder ausgewertet werden können. Mit anderen Worten können mehrere Messdaten und/oder Messergebnisse zumindest teilweise parallel von der Steuereinheit empfangen und/oder verarbeitet werden. Dies kann entsprechende Hardwareanforderungen an die Steuereinheit stellen, insbesondere hinsichtlich der Prozessorleistung und/oder des Arbeitsspeichers, um das parallele Empfangen und/oder Auswerten mehrerer Signale und/oder Daten zu ermöglichen.

Die Erfindung bietet den Vorteil, dass zumindest das Durchführen der Funktionsprüfung des Steuerelements und das Ermitteln des variablen Parameters und das Bestimmten des Einflusses auf die beabsichtigte Durchführung der Laserbearbeitung zumindest teilweise zeitgleich durchgeführt werden und somit die für den Systemtest erforderliche Zeitdauer reduziert wird. Mit anderen Worten werden ein Test und eine Kalibrierung im Rahmen des Systemtests zumindest teilweise zeitgleich durchgeführt. Somit bietet die Erfindung den Vorteil, dass ein größerer Anteil oder gar sämtliche Tests und/oder Kalibrierungen des Systemtests unmittelbar vor der Benutzung des Lasersystems durchgeführt werden können, ohne dabei ein für den Patienten und/oder Benutzer akzeptables Zeitmaß zu überschreiten.

Auch bietet die Erfindung den Vorteil, dass eine Aufteilung von Tests und/oder Kalibrierungen, die unmittelbar vor einer Bearbeitung bzw. Behandlung mittels des Laserbearbeitungssystems durchgeführt werden und anderen Tests und/oder Kalibrierungen, die zu einem anderen Zeitpunkt durchgeführt werden, nicht zwingend erforderlich ist, um die Dauer des Systemtests unmittelbar vor der Bearbeitung bzw. Behandlung, in der etwa ein Patient bereits angedockt ist, auf ein akzeptables Maß zu begrenzen.

Ferner bietet die Erfindung den Vorteil, dass die Wartezeit, welche der zu behandelnde Patient ggf. in angedocktem Zustand vor Behandlungsbeginn verweilen muss, reduziert werden kann und entsprechend die Unannehmlichkeiten für den Patienten durch das Warten verringert werden können. Außerdem kann ggf. eine Dosis der Medikamentierung für den Patienten, welche für die Durchführung der Behandlung erforderlich ist, reduziert werden, da der Zeitabstand zwischen der Verabreichung der Medikamente und dem Behandlungsbeginn verkürzt werden kann.

Optional ist das Steuerelement als Scaneinrichtung zur Strahlablenkung ausgebildet. Insbesondere kann das Steuerelement als Laserstrahl-Scanner (auch als Scaneinrichtung bezeichnet), als Aktuator, als akusto-optischer Modulator (AOM), als elektro-optischer Modulator und/oder als adaptiver Spiegel ausgebildet sein oder zumindest eines dieser Elemente umfassen. Dabei umfasst optional die Funktionsprüfung des Steuerelements einen Test der Scaneinrichtung mittels zumindest eines Steuerelementsensors oder aus besteht diesem. Ein negatives Ergebnis des Tests bzw. der Funktionsüberprüfung kann dann auf einen Fehler hindeuten, wobei das Steuerelement und/oder der Sensor für den Fehler ursächlich sein können. Um die Zuverlässigkeit der Erkennung eines Fehlers des Steuerelements zu erhöhen, können optional zwei oder mehr redundante Sensoren für die Funktionsüberprüfung des Steuerelements verwendet werden. Dies bietet somit den Vorteil, dass die korrekte Funktionsweise der Scaneinrichtung zur Strahlablenkung vor Beginn der Behandlung zuverlässig überprüft werden kann.

Optional umfasst der Test ein Ansteuern der Scaneinrichtung derart, dass die Scaneinrichtung gemäß eines Scanmusters zum Führen eines Laserstrahls entlang eines vorbestimmten Scanpfades eingestellt wird, wobei eine Einstellung der Scaneinrichtung zur jeweiligen Ablenkung des Laserstrahls durch die Scaneinrichtung mittels des zumindest einen Steuerelementsensors ermittelbar ist. Ein Scanmuster ist im Sinne der hier beschriebenen Erfindung eine Sequenz von Steuerdaten, welche der Scaneinrichtung zugeführt werden kann, um eine Sequenz von Strahlführungskonfigurationen herbeizuführen. Der Scanpfad ist hingegen eine Sequenz virtueller Fokuspositionen, die sich aus der Sequenz der zugehörigen Strahlführungskonfigurationen, d.h. aus dem Scanmuster, ergibt. Mit anderen Worten ist das Scanmuster eine Sequenz von Einstellungen für die Scaneinrichtung, welche dazu führt, dass der Fokus entlang des Scanpfades bewegbar ist. Ein virtueller Fokus, welcher auch als virtuelle Fokusposition bezeichenbar ist, ist dabei eine theoretische Position eines Laserfokus, der eine bestimmte Konfiguration der Strahlführung und insbesondere der Scaneinrichtung zugeordnet werden kann. Dabei kann die Menge der virtuellen Fokuspositionen größer sein als die Menge realer Fokuspositionen, da nicht jede Konfiguration der Scaneinrichtung notwendigerweise zu einem realen Fokus führen muss. Ein realer Fokus, welcher auch als reale Fokusposition bezeichenbar ist, ist dabei eine Position eines Laserfokus, der durch Laserstrahlung entsteht, die eine bestimmte konfigurierte Strahlführung (Optik) und insbesondere bestimmt konfigurierte Scaneinrichtung durchläuft. Eine variable Konfiguration der Strahlführung kann dazu dienen, dass jede reale Fokusposition mit mindestens einer bestimmten Konfiguration der Scaneinrichtung erreicht werden kann.

Das Führen des Laserstrahls bedeutet dabei, dass der Laserstrahl mittels des Scaneinrichtung derart abgelenkt wird, dass der virtuelle und/oder reale Fokus entlang des vorgegebenen Scanpfades geführt wird. Dabei ist es unerheblich, ob beim Einstellen der Scaneinrichtung tatsächlich ein Laserstrahl einfällt und durch die Scaneinrichtung abgelenkt wird, d.h. ob ein realer Fokus entsteht, oder ob die Einstellungen der Scaneinrichtung erfolgen, ohne dass ein Laserstrahl einfällt und demnach lediglich ein virtueller Fokus abgelenkt wird. Mit anderen Worten können die Einstellungen und das Überprüfen der Scaneinrichtung auch derart vorgenommen werden, dass dabei kein Laserstrahl tatsächlich von der Scaneinrichtung abgelenkt wird, sondern lediglich die Eignung der Scaneinrichtung zur gewünschten Ablenkung des Laserstrahls überprüft wird. Dies kann beispielsweise mittels geeigneter Sensoren an der Scaneinrichtung erfolgen. Mit anderen Worten entspricht das Führen des Laserstrahls entlang eines vorbestimmten Scanmusters dem kontrollierten Herbeiführen eines Scanpfads, d.h. einer Ortsänderung des ggf. fokussierten Laserstrahls in der Ebene oder dem Volumen, in welcher bzw. welchem das zu bearbeitende Objekt mit dem Laserstrahl beaufschlagt wird. Entsprechend können zwei verschiedene Arten von Funktionstests für die Scaneinrichtung verwendet werden. Gemäß einer ersten Art erfolgt lediglich eine Prüfung der Konfiguration der Strahlführung, d.h. der mechanischen Spiegelpositionen und der zugehörigen virtuellen Fokuspositionen. Gemäß einer zweiten Art erfolgt eine Prüfung der realen Fokuspositionen, beispielsweise durch ein optisches Erfassen und Auswerten eines konfokalen Rückreflexes des jeweiligen realen Fokus.

Optional kann das Führen des Laserstrahls auch in einer Richtung parallel zur optischen Achse erfolgen, indem etwa die Position des Fokus entlang der Strahlrichtung verändert wird, beispielsweise durch eine Änderung des Konvergenzwinkels des Laserstrahls und/oder der Position des fokussierenden Elements. Dadurch kann ein Führen des Laserstrahls bzw. Fokus entlang eines dreidimensionalen Scanpfades erfolgen. Der verursachte Scanpfad des Scanmusters kann dabei kontinuierlich ausgebildet sein, oder kann Unterbrechungen aufweisen. Auch kann der Scanpfad mehrere Teilstücke aufweisen und/oder mehrere Punkte aufweisen, auf welche der Laserstrahl entlang eines vorbestimmten Scanpfades, d.h. in einer vorbestimmten Reihenfolge, appliziert und optional fokussiert wird. Mit anderen Worten ist das Scanmuster des Tests optional derart ausgebildet, dass der zugehörige Scanpfad alle für eine vorbestimmte Laserbearbeitung durch das Laserbearbeitungssystem vorgesehenen Fokuspositionen bzw. Laserstrahlpositionen umfasst. Optional kann das vorbestimmte Scanmuster derart gewählt werden, dass das Scanmuster repräsentativ für den gesamten nutzbaren Wertebereich der Scaneinrichtung ist.

Dies bietet den Vorteil, dass der Test universell einsetzbar ist und nicht nur für einzelne geplante Behandlungen individuell erstellt werden muss. Auch kann ein Scanmuster optional derart gewählt werden, dass die erforderliche Testdauer kürzer ist, als eine etwaige beabsichtigte Behandlung. Das Testen der Scaneinrichtung bietet den Vorteil, dass die Funktionalität der Scaneinrichtung zur Ablenkung des Laserstrahls entlang des für die geplante Behandlung vorbestimmten Pfades überprüft werden kann. Das Fokussieren des Laserstrahls ist dabei optional. Demnach kann es Anwendungen geben, in denen der Laserstrahl fokussiert wird und andere Anwendungen, in denen der Laserstrahl nicht fokussiert wird. Sofern eine Fokussierung erfolgt, kann das zumindest eine fokussierende Element gemäß manchen Ausführungsformen vor der Scaneinrichtung angeordnet sein und gemäß anderen optionalen Ausführungsformen nach der Scaneinrichtung angeordnet sein. Optional wird bei der Verwendung von Femtosekundenlasern der Laserstrahl erst nach der Scaneinrichtung, d.h. nach dem Ablenken, fokussiert. Optional wird bei der Verwendung von Excimerlasern der Laserstrahl vor der Scaneinrichtung fokussiert und der konvergente Laserstrahl sodann abgelenkt.

Optional ist das Scanmuster derart ausgebildet, dass der Laserstrahl im Bereich des zum Scanmuster gehörenden Scanpfads zumindest teilweise ein den variablen Parameter charakterisierendes Referenzobjekt erfasst und der variable Parameter anhand eines daraus resultierenden Signals ermittelbar ist. Dies bietet den Vorteil, dass das Erfassen des den variablen Parameter charakterisierenden Referenzobjekts mit dem Test des Steuerelements kombiniert werden kann und entsprechend während des Durchlaufs der Funktionsüberprüfung bzw. des Tests mit abgearbeitet werden kann. Dies ist für solche Referenzobjekte möglich, welche sich in einem Bereich befinden, die für den Laserstrahl zugänglich sind, d.h. in dem Bereich liegen, die mittels des durch die Scaneinrichtung abgelenkten Laserstrahl erfasst werden kann.

Optional umfasst das Signal, welches aus dem Erfassen des Referenzobjekts durch den Laserstrahl resultiert, einen reflektierten Anteil des Laserstrahls und/oder einen gestreuten Anteil des Laserstrahls und/oder ein durch den Laserstrahl angeregtes Emissionssignal. Dabei ist das Signal durch einen Referenzobjektsensor detektierbar. So kann etwa das Referenzobjekt einen Teil des einfallenden Laserstrahls reflektieren und/oder streuen, sodass der reflektierte und/oder gestreute Anteil mittels des Referenzobjektsensors detektierbar ist. Dies kann etwa zur Prüfung der Lage der realen Fokusposition in Bezug auf ein Referenzobjekt dienen. Beispielsweise kann dies mittels einer Grenzflächendetektion erfolgen, wie etwa in WO 2008/040436 A1 beschrieben. Optional wird der Laserstrahl lediglich an solchen Stellen des vorbestimmten Scanpfades bzw. bei solchen Konfigurationen des Scanmusters durch das Laserbearbeitungssystem emittiert, an denen der Laserstrahl zumindest teilweise das Referenzobjekt erfasst. Dem steht nicht entgegen, dass optional die Laserquelle des Laserbearbeitungssystems auch dann Laserstrahlung emittiert, wenn das Laserbearbeitungssystem keinen Laserstrahl emittiert, da der Laserstrahl beispielsweise innerhalb des Laserbearbeitungssystems blockiert und/oder umgelenkt werden kann und somit zwar von der Laserquelle emittiert wird, nicht aber durch das Laserbearbeitungssystem auf einen Patienten oder ein zu bearbeitendes Objekt appliziert wird. Dies bietet den Vorteil, dass der Test bzw. die Funktionsüberprüfung auch dann durchgeführt werden kann, wenn der Patient bereits an das Laserbearbeitungssystem angedockt ist oder ein zu bearbeitendes Objekt bereits am Laserbearbeitungssystem angedockt ist, insbesondere mittels einer Prüfung der virtuellen Fokusposition(en). Dabei kann die Funktionsüberprüfung des Steuerelements zumindest teilweise auch ohne emittierten Laserstrahl erfolgen, beispielsweise indem entsprechende Sensoren bereitgestellt werden, welche die genaue Positionierung und/oder Ablenkung und/oder Auslenkung und/oder Orientierung des Steuerelements auch ohne Beaufschlagung mit einem Laserstrahl ermitteln können.

Optional wird der Laserstrahl während der Funktionsprüfung des Steuerelements mit einer solchen Leistung emittiert, welche unterhalb einer Leistungsschwelle für eine Gefährdung und/oder Bearbeitung eines Auges liegt. Die Leistungsschwelle für die Bearbeitung des Auges ist dabei jene Schwelle der Laserenergie, unterhalb welcher keine Veränderung des Materials bzw. Gewebes in Bezug auf die jeweilige Applikation erfolgt. Die Leistungsschwelle für die Gefährdung des Auges ist dabei eine Sicherheitsschwelle der Laserenergie, unterhalb welcher es zu keiner Schädigung des Auges kommt und insbesondere auch kein Kollateralschaden an anderen, nicht der Behandlung unterzogenen Teilen des Auges, wie etwa der Linse und/oder der Retina entsteht. Die Begrenzung der Leistung auf eine dieser Leistungsschwellen, bevorzugt auf die Leistungsschwelle für die Gefährdung des Auges, bietet den Vorteil, dass sich die Funktionsüberprüfung des Steuerelements zumindest teilweise auch des Laserstrahls bedienen kann, wobei ein Applizieren des Laserstrahls auf ein Auge eines Patienten und/oder ein zu bearbeitendes Objekt aufgrund der Leistung unterhalb der Leistungsschwelle unschädlich ist. Insbesondere bietet dies den Vorteil, dass der Laserstrahl während der Funktionsüberprüfung des Steuerelements dazu verwendet werden kann, um ein bereitgestelltes Referenzobjekt zu erfassen, welches den variablen Parameter charakterisiert.

Der variable Parameter kann optional ein veränderliches Element des Laserbearbeitungssystems charakterisieren, wie etwa eine Patientenandockeinheit und insbesondere ein Kontaktglas, einen Teil des optischen Systems, das Veränderungen unterworfen ist, oder einen Laserscanner und dessen Ansteuerung, welches einem Drift unterliegen kann. Optional charakterisiert der variable Parameter eine Eigenschaft des Laserbearbeitungssystems und insbesondere eine Eigenschaft eines austauschbaren Kontaktglases des Laserbearbeitungssystems. Dies kann insbesondere eine geometrische Eigenschaft des Kontaktglases sein. Der variable Parameter etwa aufgrund von kontaktglasabhängigen Abweichungen, die etwa im Rahmen von herstellungsbedingten Fertigungstoleranzen auftreten, variabel sein, wobei die Abweichungen dennoch derart ausgeprägt sein können, dass ihre Berücksichtigung zur Erreichung der gewünschten Präzision bei der Laserbehandlung bzw. Laserbearbeitung geboten erscheint. So können die variablen Parameter eines Kontaktglases beispielsweise die Stärke und/oder den Durchmesser und/oder die Zentrierung, d.h. den lateralen Versatz relativ zur optischen Achse, und/oder die Form, insbesondere den Krümmungsradius, und/oder andere geometrische Variationen des Kontaktglases oder von Teilen davon betreffen. Durch das Ermitteln des variablen Parameters und das Bestimmen des Einflusses auf die beabsichtigte Durchführung der Laserbearbeitung können somit Ungenauigkeiten und eine Reduktion der Präzision aufgrund des variablen Parameters reduziert oder gar ganz vermieden werden. Als Referenzobjekt können dabei variable Ausprägungen von Komponenten des Laserbearbeitungssystems oder des Auges an sich dienen, oder auch Markierungen und/oder Markerelemente, aus denen eine gespeicherte Information ausgelesen werden kann, wie etwa ein Strichcode oder ein Farbmarker. Als Referenzobjekt, welches durch die variable Ausprägung selbst dargestellt wird, können dabei insbesondere die (zwischen einzelnen Kontaktgläsern mit Abweichungen behafteten) Kontaktglasflächen dienen. Dazu kann der Laserstrahl bzw. der Fokus während des Tests des Laserscanners derart geführt werden, dass der Laserstrahl entlang des Scanpfades die Kontaktglasoberfläche schneidet und der dabei reflektierte und/oder gestreute Teil des Laserstrahls durch einen Referenzobjektsensor detektiert wird und von der Steuereinheit empfangen und ausgewertet werden kann. Bei der Kontaktglasoberfläche handelt es sich um eine optische Grenzfläche, die als Referenzfläche für das Patientenauge dient, welches an die Kontaktglasoberfläche angedockt ist. In angedocktem Zustand nimmt das Auge die Form dieser Grenzfläche an. Daher kann eine genaue Kenntnis der Lage dieser Grenzfläche in Bezug auf die Laserfokusposition von Bedeutung für eine genaue Bearbeitung der Hornhaut oder anderer Teile des Auges sein. Die Zuordnung der vom Referenzobjekt erfassten Signale und die zugehörigen Scannerpositionen bzw. Positionen des Laserstrahls bzw. des Fokus ermöglichen sodann eine Ermittlung der geometrischen Eigenschaften des Kontaktglases und somit des variablen Parameters. Beispielsweise kann dazu ein Verfahren verwendet werden, welches in der Offenlegungsschrift WO 2008/040436 A1 beschrieben ist. Das Scanmuster, entlang welchem der Laserstrahl während des Abtastvorgangs des Kontaktglases bewegt wird, kann dabei eine spiralförmige Scanbahn umfassen oder daraus bestehen. Beispielsweise kann die Scanbahn ähnlich oder identisch zu einer Fokustrajektorie für einen Flapschnitt ausgebildet sein. Alternativ oder zusätzlich kann ein Scan unterhalb des Scheitels der sphärischen Grenzfläche ausgeführt werden. Dabei kann eine Ebene der realen Fokuspositionen die sphärische Grenzfläche in einem Kreis schneiden. Mit anderen Worten entsteht ein konfokales Signal in Form eines Kreises, dessen Mittelpunkt mit dem Scheitelpunkt der Grenzfläche übereinstimmt und dessen Durchmesser über eine Kugelkappenform bei bekanntem Krümmungsradius der Grenzfläche die Höhe des Scheitels indiziert.

Optional kann das Kontaktglas eine oder mehrere Markierungen aufweisen, welche als Referenzobjekt dienen und Informationen über das Kontaktglas enthalten, wie etwa über die Art, Form und/oder räumliche Anordnung. Beispielsweise können die Informationen Auskunft über einen Kontaktglastyp eine Winkelausrichtung, einen formbeschreibenden Parameter und/oder eine Seriennummer geben, beispielsweise mittels eines Strichcodes. Die Markierungen können dabei insbesondere durch ihre räumliche Positionierung und/oder Orientierung im und/oder am Kontaktglas eine Information über den variablen Parameter bereitstellen. Alternativ oder zusätzlich können eine oder mehrere Referenzobjekte ausgebildet sein, welche nicht durch ihre Positionierung und/oder Orientierung Information bereitstellen, sondern durch andere Eigenschaften. Beispielsweise können solche Referenzobjekte als Marker ausgebildet sein, deren Eigenschaften bei einem Erfassen mit dem Laserstrahl durch den Referenzobjektsensor erfasst werden können. Beispielsweise können die Marker ein Lumineszenzsignal mit vorbestimmter Wellenlänge bereitstellen, sodass anhand dessen Wellenlänge bzw. Farbe eine entsprechende Information extrahiert werden kann. Beispielsweise kann die Kontaktglasgröße durch einen entsprechenden Lumineszenz-Farbcode angegeben werden.

Die Ansteuersignale für den Laserscanner können dabei so geartet sein, dass sie für die x- und y- Scanner exakt denen einer Behandlung entsprechen. Der z-Scanner wird leicht abweichend von einer Behandlung angesteuert, so dass die Kontaktglasfläche von der Fokustrajektorie sicher geschnitten wird. Die Laserstrahlung ist während des Systemtests aktiv, wird jedoch optional auf ein ungefährliches Maß (Laserklasse 1) abgeschwächt. Weitere Parameter der Laserstrahlung können während des Systemtests wie bei der eigentlichen Behandlung geregelt und/oder überwacht werden (z.B.: Pulse-picking). Als Referenzobjektsensor für den Abtastvorgang dient optional eine Konfokaloptik mit Photodiode als Sensor. Ein Teil des einfallenden Laserstrahls kann dabei zunächst von der Kontaktglasoberfläche zurück in den einfallen Laserstrahl reflektiert und über einen polarisierenden Strahlteiler aus dem Hauptpfad abgezweigt werden. Anhand des detektierten Signals kann sodann der variable Parameter ermittelt werden und für die Optimierung der Steuersignale für den Laserscanner verwendet werden, um die Genauigkeit der Behandlung zu erhöhen.

Optional betrifft der variable Parameter eine Eigenschaft eines mittels des Laserbearbeitungssystems zu behandelnden Auges, insbesondere eine veränderliche räumliche Position des Auges relativ zum Laserbearbeitungssystem. Dabei dient optional als Referenzobjekt eine Oberfläche im Auge, welche einen Teil des Laserstrahls reflektiert und/oder streut. Beispielsweise können die Iris und/oder die Netzhaut und/oder die Hornhaut oder Teile davon solch eine Oberfläche darstellen. Dies bietet den Vorteil, dass während der Funktionsüberprüfung des Steuerelements beispielsweise die Ausrichtung und/oder Positionierung des Auges, welches an das Laserbearbeitungssystem angedockt ist, ermittelt werden können.

Optional betrifft der variable Parameter eine veränderliche Eigenschaft einer Messeinheit der Scaneinrichtung, insbesondere einer Signalwandlungseinheit, wobei als Referenzobjekt zumindest ein Teil eines optischen Elements im Strahlengang des Laserbearbeitungssystems mit vorbekannter Geometrie dient. Dabei kann beispielsweise die bekannte Oberfläche des als Referenzobjekt dienenden optischen Elements, etwa die Oberfläche einer Linse im Strahlengang des Laserstrahls, vom Laserstrahl bzw. optional vom Fokus des Laserstrahls abgetastet werden und die von der Messeinheit bestimmte Form der Oberfläche mit der bekannten, tatsächlichen Form verglichen werden. Aus einer etwaigen Abweichung aufgrund des variablen Parameters können sodann Informationen für eine Optimierung von Steuersignalen für das Steuerelement, etwa für den Laserscanner bzw. die Scaneinrichtung, abgeleitet werden, um die Abweichung zumindest teilweise zu kompensieren. Ein entsprechendes Optimierungsverfahren kann auch auf andere veränderliche Elemente angewendet werden, sofern diese einen Einfluss auf die Ansteuerung des Laserscanners haben.

Optional umfasst das Verfahren ferner ein Überprüfen zumindest einer weiteren Komponente des Laserbearbeitungssystems aus der folgenden Liste: eine Shuttereinheit, eine Pulse-Picker Einheit, eine Abschwächereinheit. Dies bietet den Vorteil, dass auch einer oder mehrere dieser weiteren Tests zumindest teilweise zeitgleich mit den weiteren Tests und/oder Kalibrierungen durchgeführt werden können und auf diese Weise die Gesamtdauer eines Systemtests weiter verkürzt werden kann.

Optional umfasst das Verfahren ferner ein Überprüfen der Laserquelle, welche als gepulste Laserquelle ausgebildet sein kann, wie etwas als Femtosekundenlaser, insbesondere hinsichtlich zumindest eines der folgenden Parameter: Pulsenergie, Spitzenintensität, Pulsfrequenz, und Pulsdauer. Dies bietet den Vorteil, dass auch das Überprüfen der Laserquelle zumindest teilweise zeitgleich mit Tests und/oder Kalibrierungen des Laserbearbeitungssystems erfolgen kann und auf diese Weise die Gesamtdauer eines Systemtests weiter verkürzt werden kann.

Optional umfasst das Verfahren ferner eine Überprüfung von einer oder mehreren Sicherheitseinrichtungen des Laserbearbeitungssystems. Dies kann optional zeitlich nach dem Ermitteln des variablen Parameters aber zumindest teilweise zeitglich mit der Funktionsprüfung des Steuerelements erfolgen. Dazu kann optional ein Shutter im Strahlengang des Laserstrahls platziert werden, sodass ein Austreten des Laserstrahls vermieden werden kann. Auf diese Weise kann die Funktionalität des Shutters überprüft werden. Optional kann die verstrichene Zeit zwischen dem Aktivierungskommando zum Auslösen des Shutters und der vollständigen Blockade des Laserstrahls ermittelt und mit einem Referenzwert verglichen werden. Als Sensor kann beispielsweise eine Photodiode verwendet werden, die beispielsweise hinter dem Shutter angeordnet ist. Alternativ oder zusätzlich kann der Shutter zumindest teilweise als Spiegel ausgebildet sein, sodass er im geschlossenen Zustand den Laserstrahl auf eine Photodiode lenkt. Alternativ oder zusätzlich kann die Photodiode als nichtlinearer Sensor ausgeführt sein, um zudem die Spitzenintensität der Laserpulse ermitteln zu können.

Optional werden die beim Systemtest ermittelten Ergebnisse eines Soll-Ist-Vergleichs der Scannerpositionen mit vorgegebenen Toleranzbedingungen verglichen. Dabei kann ein Einhalten der vorgegebenen Toleranzen für die Freigabe des Laserbearbeitungssystems für eine Behandlung und/oder Bearbeitung erforderlich sein. Optional werden auch die beim Systemtest ermittelten Eigenschaften des veränderlichen Elements mit vorgegebenen Toleranzbedingungen verglichen. Auch dabei kann ein Einhalten der vorgegebenen Toleranzen für die Freigabe des Laserbearbeitungssystems für eine Behandlung und/oder Bearbeitung erforderlich sein.

Die beim Systemtest ermittelten Eigenschaften des veränderlichen Elements werden optional verwendet, um die für eine nachfolgende Laserbehandlung zu erzeugenden Scanner-Steuersignale an die ermittelten Eigenschaften anzupassen (z.B. Anpassung des Scanpatterns an die Position und/oder Form des Kontaktglases). Die Scannerbewegungen während des Systemtests überdecken optional den für den Scanner möglichen oder zu erwartenden Behandlungsbereich vollständig. Die Scannerbewegungen während des Systemtests enthalten optional zumindest Teile des möglichen Scanbereichs, die repräsentativ für ScannerBewegungen sind, die typischerweise auch bei einer Behandlung auftreten. Alternativ oder zusätzlich enthalten die Scannerbewegungen während des Systemtests Teile des möglichen Scanbereichs, die repräsentativ für Bewegungen sind, bei denen während einer Behandlung die größte Eintrittswahrscheinlichkeit für eine Fehlfunktion erwartet wird.

Optional können noch weitere Sensoren im Laserbearbeitungssystem am Systemtest beteiligt werden. So ist es gemäß optionalen Ausführungsformen möglich, den Systemtest durchzuführen, während die Laseroptik aus der Behandlungsposition ausgeschwenkt ist. Dies kann, je nach Ausführung, zu einer Transformation (z.B. Drehung) der Funktion führen, welche das Kontaktglaskoordinatensystem mit den Scanner-Ansteuerungssignalen verbindet. Wird beispielsweise beim Systemtest nun die Lage der Kontaktglasvorderfläche ermittelt, um die Scanner-Ansteuersignale für die folgende Behandlung darauf anzupassen, muss der Effekt der Ausschwenkung berücksichtigt werden. Dazu kann ein entsprechender Sensor vorteilhaft sein, der die Ausschwenkung vermisst.

Ferner können optional eine oder mehrere Eigenschaften von einem oder mehreren veränderlichen Elementen vermessen werden. Im Falle einer Kontaktglasdetektion können beispielsweise unterschiedliche Kontaktglasflächen-Parameter wie Lage, Form, Begrenzung, Oberflächeneigenschaften oder und/oder Verschmutzung ermittelt werden.

Außerdem ist optional eine iterative Ermittlung der Eigenschaften des veränderlichen Elements, d.h. des variablen Parameters, möglich. So kann die Ermittlung von variablen Parametern betreffend die Kontaktglasoberfläche die folgenden Teile beinhalten:
Teil 1: Messung der ungefähren Lage des Kontaktglases an drei Punkten.
Teil 2: Auf Basis der Ergebnisse von Teil 1 wird eine genauere Messung der Form entlang von mehreren Linien durchgeführt.

Das zu einer Kontaktglasdetektionseinheit, welche als Referenzobjektsensor dient, an einer Kontaktglasfläche reflektierte und/oder gestreute Licht kann beispielsweise auf dem Weg zur Detektionseinheit denselben Pfad durchlaufen, wie der einfallende Laserstrahl, oder einem anderen Pfad folgen. Beispielsweise kann zur Detektion des gestreuten und/oder reflektierten Anteils des Laserstrahls eine konfokale Detektion verwendet werden, wie etwa in der Offenlegungsschrift WO 2009/146906 A2 beschrieben. Im ersteren Fall kann beispielsweise ein Strahlteiler dazu verwendet werden, um das einlaufende vom reflektierten Licht zu trennen. Dieser Strahlteiler kann optional wiederum ein konstantes Teilungsverhältnis haben oder Polarisationseffekte ausnutzen. Desweitern kann dieser Strahlteiler (und evtl. zugehörige Elemente wie Wellenplatten) nach dem Systemtest optional aus dem Strahlengang entfernt werden. Anstelle einer Laserstrahlabschwächung während des Tests, kann optional auch eine Strahlfalle hinter der letzten relevanten optischen Fläche ein Austreten von intensiver Laserstrahlung verhindern.

Optional können noch weitere Parameter betreffend das Kontaktglas erfasst werden. Dabei werden oft bekannte Eigenschaften als Lagereferenz herangezogen. Dadurch kann die Detektionszeit bzw. Testdauer gering gehalten werden. Bei bekannter Oberflächenform der Kontaktglasoberfläche können beispielsweise drei Messpunkte im Raum ausreichend sein, um die genaue Lage der Kontaktglasoberfläche zu bestimmen.

Das Verfahren und die Steuereinheit sind optional auch für Excimerlaser anwendbar welche keinen definierten Laserfokus erzeugen. Dabei kann beispielsweise der korrekte Sitz eines Patienteninterfaces und/oder dessen Größe überprüft werden und/oder die Skalierung einer Messeinheit des Laserscanners. Dabei fallen dann entsprechend die Bedingungen betreffend den Laserfokus weg und werden allgemein durch den (nicht-fokussierten) Laserstrahl ersetzt. Die Detektion der Position des Referenzobjekts ist dann primär lateral möglich.

Die während des Systemtests genutzte Laserquelle muss nicht notwendiger identisch sein mit derjenigen, die für die Behandlung verwendet wird, wenngleich dies bei manchen optionalen Ausführungsformen der Fall ist.

Die Ausführung der Detektionseinheit für den Referenzobjektsensor kann dabei in unterschiedlichen Ausführungsformen variieren. Beispielsweise kann der Referenzobjektsensor eine Photodiode und/oder eine Kamera aufweisen. Im Falle einer Photodiode kann dieser ein optionaler Teil einer konfokalen Beobachtung zur Kontaktglasdetektion sein. Eine Kamera könnte außerdem Streulicht von eingelaserten Markierungen in einem Kontaktglas erkennen.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Weitere Einzelheiten und Vorteile der Erfindung sollen nun anhand der folgenden Beispiele und bevorzugten Ausführungsformen mit Bezug auf die Figuren näher erläutert werden.

Es zeigen:
- Figur 1: ein Laserbearbeitungssystem 100 gemäß einer optionalen Ausführungsform;
- Figur 2: eine Erläuterung des Zusammenwirkens mehrerer Komponenten für einen Systemtest gemäß einer optionalen Ausführungsform.

In den folgenden Figuren werden gleiche oder ähnliche Elemente in den verschiedenen Ausführungsformen der Einfachheit halber mit gleichen Bezugszeichen bezeichnet.

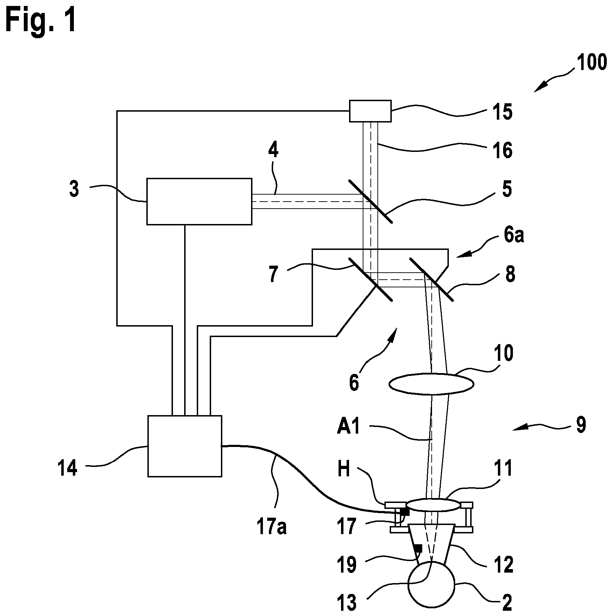

Figur 1 zeigt in einer schematischen Darstellung ein Laserbearbeitungssystem 100 gemäß einer optionalen Ausführungsform, welches als Vorrichtung für die refraktive Chirurgie eines Auges ausgebildet ist. Das Laserbearbeitungssystem 100 ist als demnach als ein Behandlungsgerät ausgebildet und dient dazu, an einem Auge 2 eines Patienten eine Fehlsichtigkeitskorrektur mittels eines Verfahrens zur refraktiven Chirurgie unter Verwendung eines Laserstrahls auszuführen. Dazu weist die Vorrichtung 100 einen Laser bzw. eine Laserquelle 3 auf, welche gepulste Laser-Strahlung abgibt. Die Pulsdauer liegt dabei z. B. im Femtosekundenbereich, und die Laserstrahlung wirkt auf die Hornhaut des Auges 2 ein, um in der Hornhaut ein Lentikel von der umliegenden Hornhaut zu separieren.

Der von der Laserquelle 3 entlang einer optischen Achse A1 abgegebene Laserstrahl bzw. Behandlungsstrahl 4 fällt dabei auf einen Strahlteiler 5, der den Laserstrahl 4 auf ein Steuerelement 6 leitet, welches als eine Scaneinrichtung 6a ausgebildet ist. Die Scaneinrichtung 6a weist gemäß der gezeigten Ausführungsform zwei Scanspiegel 7 und 8 auf, die um zueinander orthogonale Achsen drehbar sind, so dass die Scaneinrichtung 6a den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf.

Der Linse 11 ist ein Kontaktglas 12 nachgeordnet, das über eine Halterung H fest mit der Linse 11 und damit dem Laserbearbeitungssystems 100 verbunden ist. Das Kontaktglas 12 liegt an der Hornhaut des Auges 2 an. Die optische Kombination aus Kontaktglas 12 und den anderen optischen Komponenten des Laserbearbeitungssystems 100 bewirkt, dass der Behandlungsstrahl 4 in einem in der Hornhaut des Auges 2 gelegenen Fokus 13 gebündelt wird.

Die Vorrichtung 100 verfügt ferner über eine Steuereinheit 14, welche insbesondere dazu eingerichtet ist, die Scaneinrichtung 6a, den Laser 3 und die Projektionsoptik 9 zu steuern. Außerdem ist die Steuereinheit 14 dazu eingerichtet, die Funktionalität der Vorrichtung zu überwachen und zu diesem Zweck einen Systemtest zur Überprüfung der Funktionalität des Laserbearbeitungssystems 100 durchzuführen. Dazu kann die Steuereinheit 14 Elemente des Laserbearbeitungssystems 100 einem Test und/oder einer Kalibrierung unterziehen. Dabei können die Elemente der Laserbearbeitungssystem 100 von der Steuereinheit 14 zur Durchführung vorbestimmter Prozesse veranlasst werden, welche sodann über geeignete Sensoren und/oder Detektoren überwacht werden. Die Messdaten, welche dabei von den Sensoren und/oder Detektoren verwendet werden, werden sodann wieder der Steuereinheit 14 zugeführt und können von dieser für die Überprüfung der Funktionalität und für die weitere Ansteuerung des Laserbearbeitungssystems 100 verwendet werden.

Wenngleich in der gezeigten Ausführungsform nur eine Steuereinheit dargestellt ist, können gemäß anderen Ausführungsformen auch mehrere Steuereinheiten bereitgestellt werden, die die genannten Aufgaben und/oder anderweitige Aufgaben ausführen.

Das Laserbearbeitungssystem 100 weist zudem einen Referenzobjektsensor 17 auf, welcher gemäß der gezeigten Ausführungsform zur Ermittlung eines variablen Parameters betreffend das Kontaktglas 12 dient. Der Referenzobjektsensor 17 ist dabei im Bereich zwischen dem Kontaktglas 12 und der Linse 11 angeordnet und dazu eingerichtet, vom Kontaktglas reflektierte und/oder gestreute Teile des einfallenden Laserstrahls zu erfassen und die Messergebnisse über eine Kommunikationsleitung 17a an die Steuereinheit 14 zur weiteren Auswertung zu übermitteln. Gemäß anderen Ausführungsformen kann der Referenzobjektsensor aber auch an anderer Stelle angeordnet sein. Beispielsweise kann auch der Detektor 15 als konfokaler Referenzobjektsensor dienen. Als Referenzobjekt 19 kann dabei beispielsweise eine Markierung innerhalb des Kontaktglases dienen, welches einen Teil des einfallenden Laserstrahls zurückstreut und/oder reflektiert, sodass anhand des zurückgestreuten und/oder reflektierten Teils des Laserstrahls Informationen zum variablen Parameter ermittelt werden können, wie etwa über geometrische Abmessungen und/oder Orientierungen des Kontaktglases 12. Alternativ oder zusätzlich kann das Referenzobjekt 19, wenn vom Laserstrahl erfasst, ein Lumineszenzsignal emittieren, welches vom selben Referenzobjektsensor 17 erfassbar ist und durch die Steuereinheit 14 daraus eine weitere Information gewinnbar ist, wie etwa über die Größe des Kontaktglases. Dies kann beispielsweise durch die Auswertung der Wellenlänge bzw. Farbe des Lumineszenzsignals erfolgen. Die Lumineszenz kann dabei auf Fluoreszenz und/oder Phosphoreszenz beruhen.

Die Steuereinheit 14 liest weiter einen Detektor 15 des Laserbearbeitungssystems 100 aus, der von der Hornhaut zurückgestreute und/oder reflektierte Strahlung, die durch das Kontaktglas 12 hindurchtritt und den Strahlteiler 5 als Rückstrahlung 16 passiert. Dazu kann eine konfokale Abbildung der rückgestreuten Rückstrahlung 16 auf den Detektor 15 erfolgen. Auch dieser Detektor kann gemäß einer optionalen Ausführungsform als Referenzobjektsensor dienen und einen variablen Parameter betreffend das Auge 2 ermitteln. Beispielsweise kann mittels des Detektors Information über die Positionierung und/oder Orientierung des Auges 2 als (weiterer) variabler Parameter ermittelt und an die Steuereinheit 14 weitergeleitet werden. Das Referenzobjekt 19 kann durch eine Eigenschaft der Hornhaut oder aus dem Inneren des Auges 2 gebildet werden.

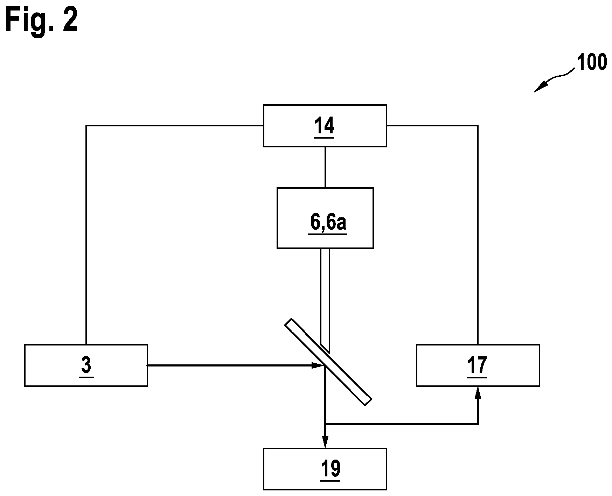

Ein Verfahren zur Durchführung eines Systemtests des Laserbearbeitungssystems 100 gemäß einer optionalen Ausführungsform wird im Folgenden anhand Figur 2 erläutert. Figur 2 zeigt in einer schematischen Darstellung das Zusammenwirken der Laserquelle 3, der Scaneinrichtung6a, des Referenzobjekts 19, des Referenzobjektsensors 17 und der Steuereinheit 14.

Dabei soll beispielsweise ein variabler Parameter, welcher das Kontaktglas 12 charakterisiert, ermittelt werden. Der variable Parameter, der im Rahmen des Systemtests ermittelt werden soll, ist dabei die genaue Lage der Cornea-Vorderseite, welche durch die exakten geometrischen Abmessungen des Kontaktglases 12 beeinflusst ist. Aufgrund der Abweichungen zwischen den verschiedenen Augen und der daraus resultierenden Änderungen des variablen Parameters ist eine genaue Ermittlung des variablen Parameters für die Erreichung eines hohen Maßes an Präzision vorteilhaft. Insbesondere bei der Verwendung von nicht-aplanierenden Kontaktgläsern können die Unterschiede zwischen verschiedenen Augen das erreichbare Maß an Präzision stark beeinflussen. Aufgrund von Fertigungstoleranzen können jedoch auch bei aplanierenden Kontaktgläsern geringe Abweichungen zwischen ansonsten gleichartigen Kontaktgläsern 12 auftreten, welche zur Erreichung eines hohen Maßes an Präzision berücksichtigt werden sollen.

Um die Dauer des Systemtests gering zu halten, wird gemäß der erläuterten Ausführungsform die Funktionsprüfung der als Steuerelement dienenden Scaneinrichtung 6 und das Ermitteln des variablen Parameters (genaue Lage der Cornea-Vorderseite; durch Kontaktglas bestimmt) zumindest teilweise zeitgleich durchgeführt. Die Funktionsprüfung der Scaneinrichtung 6a umfasst dabei ein Bewegen von Spiegeln entlang eines Scanmusters in jene Positionen, die für die geplante Behandlung erforderlich sind, und optional in noch weitere Positionen. Dazu können die Spiegel der Scaneinrichtung 6a beispielsweise mit geeigneten Winkelsensoren versehen sein. Die Messdaten der Winkelsensoren werden an die Steuereinheit übermittelt. Gleichzeitig wird zu den Zeitpunkten, an denen die Scaneinrichtung im Rahmen der Funktionsüberprüfung entlang des Scanpfades derart eingestellt ist, dass ein hindurchtretender Laserstrahl auf das im Kontaktglas befindliche Referenzobjekt 19 fällt, der Laserstrahl freigegeben, sodass das Referenzobjekt 19 vom Laserstrahl erfasst wird. Dabei ist anzumerken, dass dies während der laufenden Funktionsüberprüfung der Scaneinrichtung erfolgt und somit beide Prozesse gleichzeitig durchgeführt werden. Das vom Laserstrahl erfasste Referenzobjekt 19 sendet dabei ein Signal an den Referenzobjektsensor 17. Dieses Signal kann beispielsweise einen reflektierten und/oder gestreuten Teil des Laserstrahls beinhalten und/oder ein durch den Laserstrahl angeregtes Lumineszenzsignal. Die von dem Referenzobjektsensor 17 erfassten Messdaten werden sodann an die Steuereinheit 14 übermittelt und von dieser ausgewertet.

Somit beinhaltet die Funktionalität zum einen die Ansteuerung der Laserquelle 3 und der Scaneinrichtung 6a zur Durchführung des Systemtests, als auch das zumindest teilweise zeitgleiche Empfangen von Messdaten, welche von den Sensoren der Scaneinrichtung (bspw. Winkelsensoren der Spiegel) bei der Funktionsprüfung erfasst und an die Steuereinheit übermittelt werden, als auch das Empfangen der vom Referenzobjektsensor 17 übermittelten Messdaten. Die Steuereinheit 14 ist daher derart auszugestalten, dass diese die zeitgleiche Ausübung dieser mehreren Aufgaben gerecht wird. Insbesondere ist die Steuereinheit 14 mit einer entsprechenden Prozessorleistung als auch mit entsprechender Arbeitsspeicherkapazität auszugestalten. Die Steuereinheit 14 kann sodann anhand der empfangenen und ausgewerteten Messdaten einen Soll-Ist-Vergleich durchführen und das Ergebnis mit vorbestimmten Toleranzwerten abgleichen. Sofern das sodann von der Steuereinheit 14 ermittelte Ergebnis des Systemtests auf eine fehlerfreie Funktion des Laserbearbeitungssystems 100 hindeutet, kann die Steuereinheit das Laserbearbeitungssystem für die Behandlung freigeben. Sofern das Ergebnis des Systemtests auf keine ausreichende Funktionstüchtigkeit des Laserbearbeitungssystems hindeutet, kann die Steuereinheit 14 die Durchführung der Behandlung unterbinden und/oder einen entsprechenden Warnhinweis an den Benutzer ausgeben.

### Bezugszeichenliste

- 2: Auge
- 3: Laser
- 4: Laserstrahl
- 5: Strahlteiler
- 6: Steuerelement
- 6a: Scaneinrichtung
- 7: Scanspiegel
- 8: Scanspiegel
- 9: Projektionsoptik
- 10: Linse
- 11: Linse
- 12: Kontaktglas
- 13: Fokus
- 14: Steuereinheit
- 15: Detektor
- 16: Rückstrahlung
- 17: Referenzobjektsensor
- 17a: Kommunikationsleitung
- 19: Referenzobjekt

- 100: Laserbearbeitungssystem

- A1: optische Achse

## Patentansprüche

1. Verfahren zur Durchführung eines Systemtests eines Laserbearbeitungssystems (100), das Verfahren umfassend die Schritte:
- Durchführen einer Funktionsprüfung eines Steuerelements (6) des Laserbearbeitungssystems (100), wobei das Steuerelement (6) als Scaneinrichtung (6a) zur Strahlablenkung ausgebildet ist und die Funktionsprüfung des Steuerelements (6) einen Test der Scaneinrichtung (6a) mittels zumindest eines Steuerelementsensors umfasst oder aus diesem besteht und der Test ein Ansteuern der Scaneinrichtung (6a) derart umfasst, dass die Scaneinrichtung (6a) gemäß einem Scanmuster zum Führen eines Laserstrahls (4) entlang eines vorbestimmten Scanpfades eingestellt wird, wobei eine jeweilige Einstellung der Scaneinrichtung (6a) zur Ablenkung des Laserstrahls (4) durch die Scaneinrichtung (6a) mittels des zumindest einen Steuerelementsensors ermittelbar ist; und
- Ermitteln eines variablen Parameters und Bestimmen eines Einflusses des ermittelten variablen Parameters auf eine beabsichtigte Durchführung einer Laserbearbeitung mittels des Laserbearbeitungssystems (100);
**dadurch gekennzeichnet, dass:**
- das Durchführen der Funktionsprüfung des Steuerelements (6) und das Ermitteln des variablen Parameters zumindest teilweise zeitlich einander überlappen; und
- das Scanmuster derart ausgebildet ist, dass der Laserstrahl (4) im Bereich des Scanpfades zumindest teilweise ein den variablen Parameter charakterisierendes Referenzobjekt (19) erfasst und der variable Parameter anhand eines daraus resultierenden Signals ermittelbar ist.

2. Verfahren gemäß Anspruch 1, wobei das Scanmuster derart ausgebildet ist, dass der resultierende Scanpfad zumindest einen Teil jener Fokuspositionen umfasst, die für eine vorbestimmte Laserbearbeitung durch das Laserbearbeitungssystem (100) vorgesehenen sind.

3. Verfahren gemäß Anspruch 1 oder 2, wobei das Signal, welches aus dem Erfassen des Referenzobjekts (19) durch den Laserstrahl (14) resultiert, einen reflektierten Anteil des Laserstrahls (4) und/oder einen gestreuten Anteil des Laserstrahls (4) und/oder ein durch den Laserstrahl (4) angeregtes Emissionssignal umfasst, und wobei das Signal durch einen Referenzobjektsensor (17) detektierbar ist.

4. Verfahren gemäß einem der vorhergehenden Ansprüche, wobei der Laserstrahl (4) lediglich an solchen Stellen des vorbestimmten Scanpfades durch das Laserbearbeitungssystem (100) emittiert wird, an denen der Laserstrahl (4) zumindest teilweise das Referenzobjekt (19) erfasst.

5. Verfahren gemäß einem der vorhergehenden Ansprüche, wobei der Laserstrahl (4) während der Funktionsprüfung des Steuerelements (6) mit einer Leistung emittiert wird, welche unterhalb einer Leistungsschwelle für eine Gefährdung und/oder Bearbeitung eines Auges (2) liegt.

6. Verfahren gemäß einem der vorhergehenden Ansprüche, wobei der variable Parameter eine Eigenschaft des Laserbearbeitungssystems (100) betrifft.

7. Verfahren gemäß Anspruch 6, wobei der variable Parameter eine geometrische Eigenschaft eines austauschbaren Kontaktglases (12) des Laserbearbeitungssystems (100) charakterisiert.

8. Verfahren gemäß einem der Ansprüche 1 bis 5, wobei der variable Parameter eine Eigenschaft eines mittels des Laserbearbeitungssystems (100) zu behandelnden Auges (2) betrifft.

9. Verfahren gemäß Anspruch 8, wobei der variable Parameter eine veränderliche räumliche Position des Auges (2) relativ zum Laserbearbeitungssystem (100), und wobei als Referenzobjekt (19) eine Oberfläche im Auge (2) dient, welche einen Teil des Laserstrahls (14) reflektiert und/oder streut.

10. Verfahren gemäß einem der Ansprüche 1 bis 5, wobei der variable Parameter eine veränderliche Eigenschaft einer Messeinheit der Scaneinrichtung (6a), insbesondere einer Signalwandlungseinheit, betrifft und wobei als Referenzobjekt (19) zumindest ein Teil eines optischen Elements im Strahlengang des Laserbearbeitungssystems (100) mit vorbekannter Geometrie dient.

11. Verfahren gemäß einem der vorhergehenden Ansprüche, ferner umfassend ein Überprüfen zumindest einer weiteren Komponente des Laserbearbeitungssystem (100) aus der folgenden Liste: eine Shuttereinheit, eine Pulse-Picker Einheit, eine Abschwächereinheit.

12. Verfahren gemäß einem der vorhergehenden Ansprüche, ferner umfassend ein Überprüfen der Laserquelle (3) hinsichtlich zumindest eines Parameters der Laserquelle (3).

13. Verfahren gemäß Anspruch 12, wobei die Laserquelle (3) als eine gepulste Laserquelle ausgebildet ist und das Überprüfen der gepulsten Laserquelle (3) hinsichtlich zumindest eines der folgenden Parameter erfolgt: Pulsenergie, Spitzenintensität, Pulsfrequenz, und Pulsdauer.

14. Steuereinheit (14) für ein Laserbearbeitungssystem (100), wobei die Steuereinheit (14) dazu eingerichtet ist, im Rahmen eines Systemtests des Laserbearbeitungssystems (100)
- eine Funktionsprüfung des Steuerelements (6) des Laserbearbeitungssystems (100) durchzuführen, wobei das Steuerelement (6) als Scaneinrichtung (6a) zur Strahlablenkung ausgebildet ist und die Funktionsprüfung des Steuerelements (6) einen Test der Scaneinrichtung (6a) mittels zumindest eines Steuerelementsensors umfasst oder aus diesem besteht und der Test ein Ansteuern der Scaneinrichtung (6a) derart umfasst, dass die Scaneinrichtung (6a) gemäß einem Scanmuster zum Führen eines Laserstrahls (4) entlang eines vorbestimmten Scanpfades eingestellt wird, wobei eine jeweilige Einstellung der Scaneinrichtung (6a) zur Ablenkung des Laserstrahls (4) durch die Scaneinrichtung (6a) mittels des zumindest einen Steuerelementsensors ermittelbar ist; und
- einen variablen Parameter zu ermitteln und einen Einfluss des variablen Parameters auf eine beabsichtigte Durchführung einer Laserbearbeitung mittels des Laserbearbeitungssystems (100) zu ermitteln;
**dadurch gekennzeichnet, dass**
- die Steuereinheit (14) ferner dazu eingerichtet ist, die Funktionsprüfung des Steuerelements (6) und das Ermitteln des variablen Parameters zumindest teilweise zeitlich einander überlappend durchzuführen und daraus resultierende Messdaten und/oder Messergebnisse zumindest teilweise zeitgleich zu empfangen und/oder auszuwerten; und
- das Scanmuster derart ausgebildet ist, dass der Laserstrahl (4) im Bereich des Scanpfades zumindest teilweise ein den variablen Parameter charakterisierendes Referenzobjekt (19) erfasst und der variable Parameter anhand eines daraus resultierenden Signals ermittelbar ist.

15. Laserbearbeitungssystem (100), umfassend eine Steuereinheit (14) gemäß Anspruch 14, wobei das Laserbearbeitungssystem (100) optional als eine Vorrichtung für die refraktive Chirurgie eines Auges (2) ausgebildet ist.

## Claims

1. Method for carrying out a system test of a laser processing system (100), the method comprising the steps of:
- carrying out a functional test of a control element (6) of the laser processing system (100), wherein the control element (6) is designed as a scanning device (6a) for beam deflection and the functional test of the control element (6) comprises or consists of a test of the scanning device (6a) by means of at least one control element sensor and the test comprises controlling the scanning device (6a) in such a way that the scanning device (6a) is set in accordance with a scanning pattern for guiding a laser beam (4) along a predetermined scanning path, wherein a respective setting of the scanning device (6a) for deflecting the laser beam (4) by way of the scanning device (6a) is ascertainable by means of the at least one control element sensor; and
- ascertaining a variable parameter and determining an influence of the ascertained variable parameter on intended carrying out of laser processing by means of the laser processing system (100);
**characterized in that**:
- carrying out the functional test of the control element (6) and ascertaining the variable parameter overlap one another at least partly in time; and
- the scanning pattern is designed in such a way that the laser beam (4) in the region of the scanning path at least partly detects a reference object (19) characterizing the variable parameter, and the variable parameter is ascertainable on the basis of a signal resulting therefrom.

2. Method according to Claim 1, wherein the scanning pattern is designed in such a way that the resulting scanning path comprises at least some of those focus positions which are provided for predetermined laser processing by way of the laser processing system (100).

3. Method according to Claim 1 or 2, wherein the signal resulting from the detection of the reference object (19) by the laser beam (14) comprises a reflected portion of the laser beam (4) and/or a scattered portion of the laser beam (4) and/or an emission signal excited by the laser beam (4), and wherein the signal is detectable by a reference object sensor (17).

4. Method according to any of the preceding claims, wherein the laser beam (4) is emitted only at such locations of the predetermined scanning path through the laser processing system (100) at which the laser beam (4) at least partly detects the reference object (19).

5. Method according to any of the preceding claims, wherein the laser beam (4) is emitted during the functional test of the control element (6) with a power which is below a power threshold for endangerment and/or processing of an eye (2).

6. Method according to any of the preceding claims, wherein the variable parameter concerns a property of the laser processing system (100).

7. Method according to Claim 6, wherein the variable parameter characterizes a geometric property of an exchangeable contact glass (12) of the laser processing system (100).

8. Method according to any of Claims 1 to 5, wherein the variable parameter concerns a property of an eye (2) to be treated by means of the laser processing system (100).

9. Method according to Claim 8, wherein the variable parameter concerns a variable spatial position of the eye (2) relative to the laser processing system (100), and wherein a surface in the eye (2) which reflects and/or scatters a part of the laser beam (14) serves as reference object (19).

10. Method according to any of Claims 1 to 5, wherein the variable parameter concerns a variable property of a measuring unit of the scanning device (6a), in particular of a signal conversion unit, and wherein at least one part of an optical element in the beam path of the laser processing system (100) with previously known geometry serves as reference object (19).

11. Method according to any of the preceding claims, furthermore comprising checking at least one further component of the laser processing system (100) from the following list: a shutter unit, a pulse picker unit, an attenuator unit.

12. Method according to any of the preceding claims, furthermore comprising checking the laser source (3) with regard to at least one parameter of the laser source (3).

13. Method according to Claim 12, wherein the laser source (3) is designed as a pulsed laser source and the pulsed laser source (3) is checked with regard to at least one of the following parameters: pulse energy, peak intensity, pulse frequency, and pulse duration.

14. Control unit (14) for a laser processing system (100), wherein the control unit (14) is configured, in the context of a system test of the laser processing system (100),
- to carry out a functional test of the control element (6) of the laser processing system (100), wherein the control element (6) is designed as a scanning device (6a) for beam deflection and the functional test of the control element (6) comprises or consists of a test of the scanning device (6a) by means of at least one control element sensor and the test comprises controlling the scanning device (6a) in such a way that the scanning device (6a) is set in accordance with a scanning pattern for guiding a laser beam (4) along a predetermined scanning path, wherein a respective setting of the scanning device (6a) for deflecting the laser beam (4) by way of the scanning device (6a) is ascertainable by means of the at least one control element sensor; and
- to ascertain a variable parameter and to ascertain an influence of the variable parameter on intended carrying out of laser processing by means of the laser processing system (100);
**characterized in that**
- the control unit (14) is furthermore configured to carry out the functional test of the control element (6) and the ascertaining of the variable parameter in a manner overlapping one another at least partly in time and to receive and/or to evaluate resultant measurement data and/or measurement results at least partly simultaneously; and
- the scanning pattern is designed in such a way that the laser beam (4) in the region of the scanning path at least partly detects a reference object (19) characterizing the variable parameter, and the variable parameter is ascertainable on the basis of a signal resulting therefrom.

15. Laser processing system (100), comprising a control unit (14) according to Claim 14, wherein the laser processing system (100) is optionally designed as an apparatus for refractive surgery on an eye (2).

## Revendications

1. Procédé de réalisation d'un test de système d'un système de traitement par laser (100), le procédé comprenant les étapes :
- réalisation d'un essai de fonction d'un élément de commande (6) du système de traitement par laser (100), l'élément de commande (6) étant conçu comme installation de balayage (6a) pour la déviation d'un faisceau et l'essai de fonction de l'élément de commande (6) comprenant un test de l'installation de balayage (6a) au moyen d'au moins un capteur d'élément de commande ou étant constitué par celui-ci et le test comprenant une commande de l'installation de balayage (6a) de telle sorte que l'installation de balayage (6a) est réglée selon un motif de balayage pour le guidage d'un faisceau laser (4) le long d'un chemin de balayage défini au préalable, un réglage respectif de l'installation de balayage (6a) pour la déviation du faisceau laser (4) par l'installation de balayage (6a) pouvant être déterminé au moyen dudit au moins un capteur d'élément de commande ; et
- identification d'un paramètre variable et détermination d'une influence du paramètre variable identifié sur une réalisation envisagée d'un traitement par laser au moyen du système de traitement par laser (100) ;
**caractérisé en ce que** :
- la réalisation de l'essai de fonction de l'élément de commande (6) et l'identification du paramètre variable se chevauchent au moins partiellement dans le temps ; et
- le motif de balayage est conçu de telle sorte que le faisceau laser (4) identifie dans la zone du chemin de balayage au moins partiellement un objet de référence (19) caractérisant le paramètre variable et le paramètre variable peut être identifié à l'aide d'un signal qui en résulte.

2. Procédé selon la revendication 1, le motif de balayage étant conçu de telle sorte que le chemin de balayage résultant comprend au moins une partie des positions de focalisation qui sont prévues pour un traitement par laser défini au préalable par le système de traitement par laser (100).

3. Procédé selon la revendication 1 ou 2, le signal qui résulte de l'identification de l'objet de référence (19) par le faisceau laser (14) comprenant une partie réfléchie du faisceau laser (4) et/ou une partie diffusée du faisceau laser (4) et/ou un signal d'émission excité par le faisceau laser (4), le signal étant détectable par un capteur (17) d'objet de référence.

4. Procédé selon l'une des revendications précédentes, le faisceau laser (4) étant émis par le système de traitement par laser (100) uniquement en des endroits du chemin de balayage défini au préalable où le faisceau laser (4) identifie au moins partiellement l'objet de référence (19).

5. Procédé selon l'une des revendications précédentes, le faisceau laser (4) étant émis, pendant l'essai de fonction de l'élément de commande (6), avec une puissance qui se situe sous un seuil de puissance pour un risque et/ou un traitement d'un œil (2).

6. Procédé selon l'une des revendications précédentes, le paramètre variable concernant une propriété du système de traitement par laser (100).

7. Procédé selon la revendication 6, le paramètre variable caractérisant une propriété géométrique d'un verre de contact (12) remplaçable du système de traitement par laser (100).

8. Procédé selon l'une des revendications 1 à 5, le paramètre variable concernant une propriété d'un œil (2) à traiter au moyen du système de traitement par laser (100).

9. Procédé selon la revendication 8, le paramètre variable concernant une position spatiale modifiable de l'œil (2) par rapport au système de traitement par laser (100) et une surface dans l'œil (2) qui réfléchit et/ou diffuse une partie du faisceau laser (14) servant d'objet de référence (19).

10. Procédé selon l'une des revendications 1 à 5, le paramètre variable concernant une propriété modifiable d'une unité de mesure de l'installation de balayage (6a), en particulier une unité de conversion de signal, et au moins une partie d'un élément optique dans la trajectoire du faisceau du système de traitement par laser (100) présentant une géométrie connue au préalable servant d'objet de référence (19).

11. Procédé selon l'une des revendications précédentes, comprenant en outre une vérification d'au moins un autre composant du système de traitement par laser (100) de la liste suivante : une unité d'obturateur, une unité de sélecteur d'impulsion, une unité d'atténuation.

12. Procédé selon l'une des revendications précédentes, comprenant en outre une vérification de la source laser (3) en ce qui concerne au moins un paramètre de la source laser (3).

13. Procédé selon la revendication 12, la source laser (3) étant conçue comme une source laser pulsée et la vérification de la source laser pulsée (3) ayant lieu en ce qui concerne au moins l'un des paramètres suivants : énergie d'impulsion, intensité de pointe, fréquence d'impulsion et durée d'impulsion.

14. Unité de commande (14) pour un système de traitement par laser (100), l'unité de commande (14) étant conçue, dans le cadre d'un test de système du système de traitement par laser (100), pour
- la réalisation d'un essai de fonction de l'élément de commande (6) du système de traitement par laser (100), l'élément de commande (6) étant conçu comme installation de balayage (6a) pour la déviation d'un faisceau et l'essai de fonction de l'élément de commande (6) comprenant un test de l'installation de balayage (6a) au moyen d'au moins un capteur d'élément de commande ou étant constitué par celui-ci et le test comprenant une commande de l'installation de balayage (6a) de telle sorte que l'installation de balayage (6a) est réglée selon un motif de balayage pour le guidage d'un faisceau laser (4) le long d'un chemin de balayage défini au préalable, un réglage respectif de l'installation de balayage (6a) pour la déviation du faisceau laser (4) par l'installation de balayage (6a) pouvant être déterminé au moyen dudit au moins un capteur d'élément de commande ; et
- l'identification d'un paramètre variable et l'identification d'une influence du paramètre variable sur une réalisation envisagée d'un traitement par laser au moyen du système de traitement par laser (100) ; **caractérisée en ce que**
- l'unité de commande (14) est en outre conçue pour réaliser l'essai de fonction de l'élément de commande (6) et l'identification du paramètre variable au moins partiellement en se chevauchant dans le temps et pour recevoir et/ou évaluer au moins partiellement en même temps les données de mesure et/ou les résultats de mesure qui en résultent ; et
- le motif de balayage est conçu de telle sorte que le faisceau laser (4) identifie dans la zone du chemin de balayage au moins partiellement un objet de référence (19) caractérisant le paramètre variable et le paramètre variable peut être identifié à l'aide d'un signal qui en résulte.

15. Système de traitement par laser (100), comprenant une unité de commande (14) selon la revendication 14, le système de traitement par laser (100) étant éventuellement conçu comme un dispositif pour la chirurgie réfractive d'un œil (2).
